# FASCICULE DE BREVET EUROPEEN

(11) **EP 1 962 788 B1**
(45) Date de publication et mention de la délivrance du brevet: **21.11.2012**
(21) Numéro de dépôt: 06848723.0
(22) Date de dépôt: 15.12.2006
(51) Int. Cl.: A61K 8/37, A61K 8/68, A61K 8/44, A61K 8/49, A61Q 17/04, A61Q 19/02

(54) **Utilisation de céramides et de dérivés d'acide salicylique pour dépigmenter la peau**
Verwendung von Cermiden und Salicylsäure-Derivativen zur Depigmentierung der Haut
Use of ceramides and salicylic acid derivatives for depigmenting the skin

(30) Priorité: 16.12.2005 FR 0553911; 21.12.2005 US 752007 P
(43) Date de publication de la demande: 03.09.2008
(73) Titulaire: L'Oréal, 75008 Paris (FR)
(72) Inventeur: PELLETIER, Pascale, F-92160 Antony (FR); MARION, Catherine, F-92160 Antony (FR)
(74) Mandataire: Kromer, Christophe
(86) Numéro de dépôt international: PCT/FR2006/051370
(87) Numéro de publication internationale: WO 2007/071875

(56) Documents cités:
- EP-A- 0 500 437
- EP-A- 0 647 617
- EP-A- 0 662 319
- EP-A- 0 919 226
- EP-A1- 1 552 816
- WO-A-2004/045573
- WO-A-2005/063688
- FR-A1- 2 732 594
- DATABASE WPI Week 200509 Derwent Publications Ltd., London, GB; AN 2005-076602 XP002400378 & JP 2005 002021 A (KAO CORP) 6 janvier 2005 (2005-01-06)
- GOLDSCHMIDT: "Ceramide IIIA" THE ART OF FORMULATING, [Online] 2004, XP002397987 Extrait de l'Internet: URL:http://www.archive.org/web/20040511031 654/centerchem.com/pdfs/COSMETICS+CATALOGU E>
- DONG-SEOK KIM ET AL: "Delayed ERK activation by ceramide reduces melanin synthesis in human melanocytes" CELLULAR SIGNALING, vol. 14, 2002, pages 779-785, XP002397818 cité dans la demande

## Description

La présente invention concerne un procédé de dépigmentation de la peau utilisant des céramides particuliers.

La couleur de la peau humaine est fonction de différents facteurs et notamment des saisons de l'année, de la race et du sexe; elle est principalement déterminée par la nature et la concentration de mélanine produite par les mélanocytes. Les mélanocytes sont les cellules spécialisées qui, par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestro-progestative, les hyperpigmentations localisées par hyperactivité et prolifération mélanocytaire bénigne, telles que les taches pigmentaires séniles dites lentigo actiniques, les hyperpigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir à une cicatrice donnant à la peau un aspect plus blanc), à défaut de pouvoir repigmenter la peau lésée, on achève de dépigmenter les zones de peu normale résiduelle pour donner à l'ensemble de la peau une teinte blanche homogène.

Le mécanisme de formation de la pigmentation de la peau, c'est-à-dire de la formation de la mélanine est particulièrement complexe et fait intervenir schématiquement les principales étapes suivantes :
**Tyrosine ---> Dopa ---> Dopaquinone ---> Dopachrome ---> Mélanine**

La tyrosinase (monophénol dihydroxyl phénylalanine : oxygen oxydo-reductase EC 1.14.18.1) est l'enzyme essentielle intervenant dans cette suite de réactions. Elle catalyse notamment la réaction de transformation de la tyrosine en Dopa (di-hydroxyphénylalanine) grâce à son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante si elle agit directement sur la vitalité des mélanocytes épidermiques où se déroule la métanogénèse, et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.

Les substances les plus utilisées en tant que dépigmentants sont plus particulièrement l'hydroquinone et ses dérivés, en particulier ses éthers tels que le mono-méthyléther et le monoéthyléther d'hydroquinone. Ces composés, bien qu'ils présentent une efficacité certaine, ne sont malheureusement pas exempts d'effets secondaires du fait de leur toxicité, ce qui peut rendre leur emploi délicat, voire dangereux. Cette toxicité provient de ce qu'ils interviennent sur des mécanismes fondamentaux de la mélanogénèse en tuant des cellules qui risquent alors de perturber leur environnement biologique et qui par conséquent obligent la peau à les évacuer en produisant des toxines.

Ainsi, l'hydroquinone est un composé particulièrement irritant et cytotoxique pour le mélanocyte, dont le remplacement, total ou partiel a été envisagé par de nombreux auteurs.

On a ainsi cherché des substances qui n'interviennent pas dans le mécanisme de la mélanogénèse mais qui agissent en amont sur la tyrosinase en empêchant son activation et sont de ce fait beaucoup moins toxiques. On utilise couramment comme inhibiteur de l'activation de la tyrosinase l'acide kojique qui complexe le cuivre présent dans le site actif de cette enzyme. Malheureusement, ce composé est instable en solution, ce qui complique quelque peu la fabrication de la composition.

JP-A-2005-002021 décrit des compositions cosmétiques dépigmentante comprenant le céramide type 2 de la société Séderma ou le céramide III de la société Cosmo Pharm.

Dans une documentation de la société DEGUSSA GOLDSCHMIDT Personnal care relative au Ceramide IIIA
(http://www.archive.org/web/20040511031654/centerchem.com/pdfs/COSMETICS+CATALOGUE> ) il est décrit l'emploi de Céramide IIIA dans des compositions dépigmentantes. WO 2005/063688 A décrit des compositions dépigmentantes contenant des dérivés de sphingollipid-rucinol.

WO 2004/045573 décrit une composition dépigmentante contenant le N-acétylphytosphingosine.

EP-A-0919226 décrit une composition dépigmentante comprenant un dérivé salicylate de phytosphingosine.

EP-A-0662319 décrit des compositions cosmétiques comprenant la N-oléoyldihydrosphingosine et un actif antivieillissement tel que l'acide n-octanoyl-5-salicylique.

Il subsiste le besoin d'un nouveau procédé de dépigmentation de la peau humaine à action aussi efficace que ceux connus, mais n'ayant pas leurs inconvénients, c'est-à-dire qui soit non irritant, non toxique et/ou non allergisant pour la peau.

A cet égard la Demanderesse a de manière surprenante et inattendue découvert que certains composés céramides associés à des composés d'acide salicylique-présentaient une bonne activité dépigmentante.

De façon plus précise, l'invention a donc pour objet un procédé cosmétique pour dépigmenter et/ou éclaircir une peau présentant des taches de pigmentation comprenant l'application sur la peau d'une composition comprenant, dans un milieu physiologiquement acceptable, au moins un composé de formule (I) suivante :

R₁-CHOH-CH(NH-COR₂)(CH₂OH) (I)

dans laquelle R₁ désigne un radical alkyle en C₁₁ à C₂₁, R₂ désigne un radical hydrocarboné en C₁₁-C₁₉, linéaire, éventuellement hydroxylé et le groupe hydroxyle étant en position alpha du carbonyle, et pouvant comporter une ou plusieurs insaturations éthyléniques, notamment une ou deux insaturations éthyléniques, et au moins un agent desquamant choisis parmi l'acide salicylique et ses dérivés de formule (II) décrits ci-après. Le procédé convient notamment pour éliminer les taches pigmentaires brunâtres et/ou les taches de sénescence, et/ou pour éclaircir la peau brunie.

Les composés de formule (I) utilisés selon l'invention permettent de dépigmenter et/ou d'éclaircir efficacement la peau d'êtres humains. Ils sont notamment destinés à être appliqués sur la peau d'individus présentant des taches de pigmentation brunâtres, des taches de sénescence, ou sur la peau d'individus désirant combattre l'apparition d'une couleur brunâtre provenant de la mélanogénèse, par exemple à la suite d'une exposition aux rayonnements ultra-violet.

Pour les composés de formule (I), de préférence R₁ désigne un radical hydrocarboné en C₁₃-C₁₉ ; R₂ désigne un radical hydrocarboné en C₁₃-C₁₉ linéaire, éventuellement hydroxylé, et pouvant comporter une ou plusieurs insaturations éthyléniques.

Préférentiellement, R₁ désigne un radical hydrocarboné en C₁₃-C₁₇ ; R₂ désigne un radical hydrocarboné en C₁₃-C₁₉ linéaire , éventuellement hydroxylé, et pouvant comporter une ou plusieurs insaturations éthyléniques.

Avantageusement, R₁ désigne un radical hydrocarboné en C₁₃-C₁₇ ; R₂ désigne soit un radical hydrocarboné en C₁₃-C₁₉ linéaire et pouvant comporter une ou plusieurs insaturations éthyléniques, soit un radical hydrocarboné saturé en C₁₃-C₁₉ linéaire hydroxylé, le groupe hydroxyle étant en position alpha du carbonyle.

Comme composés de formule (I) particulièrement préférés, on peut utiliser la N-oléoyldihydrosphingosine et la N-2-hydroxypalmitoyldihydrosphingosine.

Les composés de formule (I) utilisés sont connus de l'état de la technique, notamment dans les demandes EP-A-500437 et EP-A-647617.

L'article « Delayed ERK activation by ceramide reduces melanin synthesis in human melanocytes » de Dong-Seok Kim et al, Cellular Signaling, 14 (2002) p 779-785 décrit que le N-acétyl-D-erythro-sphingosine a un effet sur l'activité de tyrosinase des mélanocytes de la peau humaine contrairement à la sphingosine-1-phosphate qui n'a pas d'effet.

La société COSMOFERM commercialise le N-linoleoyl-phytosphingosine sous le nom de Ceramide IIIA^{®} et qui est connu pour avoir une action dépigmentante sur la peau.

Le terme alkyle, dans le cadre de la présente invention, signifie une chaîne hydrocarbonée saturée ou insaturée. Parmi les groupes alkyle convenant à la mise en oeuvre de l'invention, on peut notamment citer les groupes.

La composition utilisée selon l'invention comprend, dans un milieu physiologiquement acceptable, au moins un céramide répondant à la formule (I) telle que définie ci-dessus et au moins un agent desquamant de formule (II) telle que definie ci-dessous.

En particulier la composition est adaptée à une application topique sur la peau. Le milieu physiologiquement acceptable sera préférentiellement un milieu cosmétiquement ou dermatologiquement acceptable, c'est-à-dire sans odeur, couleur ou aspect désagréable, et qui ne génère pas de picotement, tiraillement ou rougeur inacceptable pour l'utilisateur.

Par milieu physiologiquement acceptable, on comprend un milieu compatible avec les matières kératiniques d'êtres humains comme la peau, les muqueuses, les ongles, le cuir chevelu et/ou les cheveux.

La composition utilisée selon l'invention peut être destinée à une application cosmétique ou pharmaceutique, particulièrement dermatologique. De préférence la composition selon l'invention est destinée à une application cosmétique.

Le céramide de formule (I) peut être présent dans la composition utilisée selon l'invention en une teneur allant de 0,001% à 10% en poids, de préférence de 0,01 % à 5% en poids, notamment de 0,1 à 2% en poids, par rapport au poids total de la composition.

La composition peut alors comprendre tous les constituants usuellement employés dans l'application envisagée.

On peut notamment citer l'eau, les solvants, les huiles d'origine minérale, animale et/ou végétale, les cires, les pigments, les charges, les tensioactifs, les actifs cosmétiques ou dermatologiques, les filtres UV, les polymères, les gélifiants, les conservateurs.

Bien entendu l'homme du métier veillera à choisir ce ou ces éventuels composés complémentaires, et/ou leur quantité, de manière telle que les propriétés avantageuses des composés selon l'invention ne soient pas, ou substantiellement pas, altérées par l'adjonction envisagée.

La composition utilisée selon l'invention peut se présenter sous toutes les formes galéniques normalement utilisées dans les domaines cosmétique et dermatologique; elle peut être notamment sous forme d'une solution aqueuse, hydroalcoolique, éventuellement gélifiée, d'une dispersion du type lotion éventuellement biphasée, d'une émulsion huile-dans-eau ou eau-dans-huile ou multiple, d'un gel aqueux, d'une dispersion d'huile dans une phase aqueuse à l'aide de sphérules, ces sphérules pouvant être des nanoparticules polymériques telles que les nanosphères et les nanocapsules ou, mieux, des vésicules lipidiques de type ionique et/ou non-ionique.

Lorsque la composition est une émulsion, la proportion de la phase grasse peut aller de 5 à 80 % en poids, et de préférence de 5 à 50 % en poids par rapport au poids total de la composition. Les huiles, les émulsionnants et les éventuels coémulsionnants utilisés dans la composition sous forme d'émulsion sont choisis parmi ceux classiquement utilisés dans le domaine considéré. L'émulsionnant et le coémulsionnant sont présents, dans la composition, en une proportion pouvant aller de 0,3 à 30 % en poids, et de préférence de 0,5 à 20 % en poids par rapport au poids total de la composition.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau sous forme d'aérosol. Elle peut également se présenter sous forme solide, et par exemple sous forme de stick.

Dans un aspect avantageux de l'invention, les compositions utilisées peuvent comporter en plus au moins un agent apaisant, et/ou au moins agent photoprotecteur organique et/ou au moins un agent photoprotecteur minéral.

Par "agent desquamant", on entend :
l'acide salicylique et ses dérivés (dont l'acide n-octanoyl 5-salicylique de formule (II) suivante.

L'agent desquamant peut être présent dans la composition pour l'utilisation selon l'invention en une teneur allant de 0,1 à 10 % en poids, par rapport au poids total de la composition, de préférence allant de 0,1 à 5 % en poids, et préférentiellement allant de 0,5 % à 2 % en poids.

Les dérivés d'acide salicylique mentionnés précédemment sont les composés de formule (II) suivante : 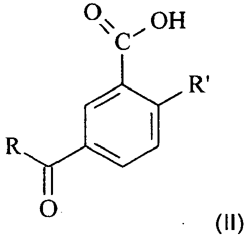 dans laquelle :
- le radical R désigne une chaîne aliphatique ayant de 2 à 22 atomes de carbone, saturée, linéaire, ramifiée ou cyclique ; une chaîne insaturée ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 7 atomes de carbone; lesdits groupes pouvant être substitués par un ou plusieurs substituants, identiques ou différents, choisis parmi (a) les atomes d'halogène (b) le groupe trifluorométhyle, (c) des groupes hydroxyle sous forme libre ou estérifiée par un acide ayant de 1 à 6 atomes de carbone ou (d) une fonction carboxyle sous forme libre ou estérifiée par un alcool inférieur ayant de 1 à 6 atomes de carbone ;
- R' est un groupe hydroxyle ;
- ainsi que leurs sels issus d'une base minérale ou organique.

De manière préférentielle , le radical R désigne une chaîne aliphatique saturée, linéaire, ramifiée ou cyclique contenant de 3 à 11 atomes de carbone ; une chaîne insaturée contenant de 3 à 17 atomes de carbone et comprenant une ou plusieurs doubles liaisons conjuguées ou non ; lesdites chaînes hydrocarbonées pouvant être substituées par un ou plusieurs substituants, identiques ou différents, choisis parmi (a) les atomes d'halogène (b) le groupe trifluorométhyle, (c) des groupes hydroxyle sous forme libre ou estérifiée par un acide ayant de 1 à 6 atomes de carbone ou (d) une fonction carboxyle sous forme libre ou estérifiée par un alcool inférieur ayant de 1 à 6 atomes de carbone ;
- ainsi que leurs sels obtenus par salification par une base minérale ou organique.

Les composés plus particulièrement préférés sont ceux dans lesquels le radical R est un groupement alkyle en C₃-C₁₁.

Parmi les composés de formule (I) particulièrement préférés, on peut citer :
l'acide n-octanoyl-5-salicylique (ou acide capryloyl salicylique) ; l'acide n-décanoyl -5-salicylique ; l'acide n-dodécanoyl-5-salicylique ; l'acide n-heptyloxy-5-salicylique et leurs sels correspondants.

On utilisera plus particulièrement l'acide n-octanoyl-5-salicylique.

Les sels des composés de formule (II) peuvent être obtenus par salification par une base minérale ou organique. A titre d'exemple de base minérale, on peut citer les hydroxydes de métal alcalin ou alcalino-terreux comme l'hydroxyde de sodium, l'hydroxyde de potassium ou l'ammoniaque.

Parmi les bases organiques, on peut citer les amines et les alcanolamines. Les sels quaternaires comme ceux décrits dans le brevet FR 2 607 498 sont particulièrement intéressants.

Les composés de formule (II) utilisables selon l'invention sont décrits dans les brevets US 6,159,479 et US 5,558,871, FR 2,581,542, FR 2 607 498, US4,767,750, EP 378,936, US 5,267,407, US 5,667,789, US 5,580,549, EP-A-570,230.

On utilise avantageusement un composition contenant la N-oléoyldihydrosphingosine et l'acide n-octanoyl-5-salicylique. Une telle composition présente une bonne performance de dépigmentation de la peau comme le montre l'exemple 4 comparatif décrit ci-après.

On peut également utiliser une composition comprenant la N-2-hydroxypalmitoyldihydrosphingosine et l'acide n-octanoyl-5-salicylique.

Comme agents apaisants utilisables dans la composition utilisée selon l'invention, on peut citer : les triterpènes pentacycliques et les extraits de plantes (ex : Glycyrrhiza glabra) en contenant comme l'acide β-glycyrrhétinique et ses sels et/ou ses dérivés (l'acide glycyrrhétinique monoglucuronide, le stearyl glycyrrhetinate, l'acide 3- stéaroyloxy glycyn-hetique), l'acide ursolique et ses sels, l'acide oléanolique et ses sels, l'acide bétulinique et ses sels, un extrait de Paeonia suffruticosa et / ou lactiflora, les sels de l'acide salicylique et en particulier le salicylate de zinc, les phycosaccharides de la société Codif, un extrait de Laminaria saccharina, l'huile de Canola, le bisabolol et les extraits de camomille, l'allantoïne, le Sépivital EPC (diesterphosphorique dé vitamine E et C) de Seppic, les huiles insaturées en oméga 3 telles que les huiles de rosier musca, de cassis, d'ecchium, de poisson, des extraits de plancton, la capryloyl glycine, le Seppicalm VG (sodium palmitoylproline et nymphea alba) de Seppic, un extrait du Pygeum, un extrait de Boswellia serrata, un extrait de Centipeda cunnighami, un extrait d'Helianthus annuus, un extrait de Linum usitatissimum, les tocotrienols, les extraits de Cola nitida, le piperonal, un extrait de clou de girofle, un extrait d'Epilobium Angustifolium, l'aloe vera, un extrait de Bacopa moniera, les phytostérols, la cortisone, l'hydrocortisone, l'indométhacine et la beta méthasone.

Les agents photoprotecteurs organiques sont notamment choisis parmi les anthranilates ; les dérivés cinnamiques ; les dérivés de dibenzoylméthane ; les dérivés salicyliques, les dérivés du camphre ; les dérivés de triazine tels que ceux décrits dans les demandes de brevet US 4367390, EP863145, EP517104, EP570838, EP796851, EP775698, EP878469, EP933376, EP507691, EP507692, EP790243, EP944624 ; les dérivés de la benzophénone ; les dérivés de β,β-diphénylacrylate ; les dérivés de benzotriazole ; les dérivés de benzalmalonate ; les dérivés de benzimidazole ; les imidazolines ; les dérivés bis-benzoazolyle tels que décrits dans les brevets EP669323 et US 2,463,264; les dérivés de l'acide p-aminobenzoïque (PABA) ; les dérivés de méthylène bis-(hydroxyphényl benzotriazole) tels que décrits dans s les demandes US5,237,071, US5,166,355, GB2303549, DE 197 26 184 et EP893119 ; et les polymères filtres et silicones filtres tels que ceux décrits notamment dans la demande WO-93/04665 ; les dimères dérivés d'α-alkylstyrène tels que ceux décrits dans la demande de brevet DE19855649.

Les agents photoprotecteurs inorganiques sont choisis parmi des pigments ou bien encore des nanopigments (taille moyenne des particules primaires: généralement entre 5 nm et 100 nm, de préférence entre 10 nm et 50 nm) d'oxydes métalliques enrobés ou non comme par exemple des nanopigments d'oxyde de titane (amorphe ou cristallisé sous forme rutile et/ou anatase), de fer, de zinc, de zirconium ou de cérium qui sont tous des agents photoprotecteurs UV bien connus en soi. Des agents d'enrobage classiques sont par ailleurs l'alumine et/ou le stéarate d'aluminium. De tels nanopigments d'oxydes métalliques, enrobés ou non enrobés, sont en particulier décrits dans les demandes de brevets EP518772 et EP518773.

Les agents photoprotecteurs sont généralement présents dans la composition selon l'invention dans des proportions allant de 0,1 à 20 % en poids par rapport au poids total de la composition, et de préférence allant de 0,2 à 15 % en poids par rapport au poids total de la composition.

Les exemples qui suivent illustrent l'invention.

### Exemple 1 : Mise en évidence de l'activité sur la mélanogénèse.

Un test biologique a mis en évidence l'activité dépigmentante des 2 composés suivants : la N-oléoyldihydrosphingosine et la N-2-hydroxypalmitoyldihydrosphingosine.

L'effet modulateur sur la mélanogénèse des composés testé a été mesuré selon la méthode décrite dans le brevet FR-A-2734825 , ainsi que dans l'article de R.Schmidt, P. Krien et M. Régnier, Anal. Bichem., 235(2), 113-18,1996. Ce test est réalisé sur coculture de kératinocytes et de mélanocytes.

Pour le composé testé, il a été déterminé l'activité inhibitrice sur la synthèse de la mélanine, en estimant le rapport de l'incorporation de thiouracile à l'incorporation de leucine, rapporté à 100 % du témoin (le témoin correspond au test réalisé sans composé à testé). Les valeurs des IC50 (concentration pour laquelle 50 % de la synthèse de la mélanine est inhibée) ont été déterminées.

On a également effectué le test avec l'arbutine et le lucinol (2-butyl benzène 1,3-diol) qui sont des composés dépigmentants connus.

Les résultats sont rassemblés dans le tableau suivant :

| | IC50 (µM) |
|---|---|
| N-oléoyldihydrosphingosine | 0,1 |
| N-2-hydroxypalmitoyldihydrosphingosine | 0,1 |
| Arbutine | 209 |
| Lucinol | 2 |

Le 2 composés testés sont bien efficaces pour inhiber la mélanogénèse.

### Exemple 2 comparatif

On a préparé une composition dépigmentante contenant les ingrédients suivants :

| | | |
|---|---|---|
| Triéthanolamine | | 0,5 g |
| Stéarate de sucrose | | |
| (Tegosoft^{®} PSE 141 G de chez Goldschmidt) | | 3 g |
| Acide myristique | | 0,03 g |
| N-2-hydroxypalmitoyldihydrosphingosine | | 1 g |
| Acide palmitique | | 0,44 g |
| Acide stéarique | | 0,53 g |
| Huile de noyau d'abricot | | 20 g |
| Sesquistéarate de méthyl glucose | | |
| (Glucate^{®} SS de chez Noveon) | | 1 g |
| Conservateurs | | qs |
| Carbomer | | 0,5 g |
| Eau | qsp | 100 g |

La composition appliquée sur une peu présentant des taches de pigmentation permet après plusieurs applications d'estomper les taches.

### Exemple 3 comparatif :

On prépare une composition dépigmentante contenant les ingrédients suivants :

| | | |
|---|---|---|
| - eau | qsp | 100 g |
| - glycérine | | 3 g |
| - conservateurs | qs | |
| - Sel disodique de l'acide éthylène diamine tétracétique | | 0,1 g |
| - gomme de xanthane | | 0,2 g |
| - polydiméthylsiloxane | | |
| - (Fluid DC 200 10 cst de chez DOW CORNING) | | 3 g |
| - conservateur | | 0,05 g |
| - isoparrafine hydrogénée (Parleam de chez NOF Corporation) | | 3 g |
| - vaseline blanche | | 1 g |
| - Mélange de monostéarate de glycéryle et de stéarate de polyéthylèneglycol (100 OE) ( Simulsol 165 de la société SEPPIC) | | 1,4 g |
| - alcool cétylique | | 1 g |
| - N-olédyldihydrosphingosine | | 0,01 g |
| - Octyldodécanol | | 1 g |
| - Polysaccharide à 1 % dans l'eau (FUCOGEL 1000 de la société SOLABIA) | | 1 g |
| - Copolymère acrylamide/acrylamido 2-méthyl propane sulfonate de sodium en émulsion inverse à 40 % dans isoparaffine/eau ( Sépigel 305 de chez SEPPIC) | | 2,4 g |
| - Hyaluronate de sodium | | 0,01 g |
| - eau | | 7 g |

La composition appliquée sur une peau présentant des taches de pigmentation permet après plusieurs applications d'estomper les taches.

### Exemple 4 :

On a préparé une composition 4a selon l'invention contenant l'association de N-oléoyldihydrosphingosine et de l'acide n-octanoyl 5-salicylique, une composition 4b ne faisant pas partie de l'invention contenant la N-oléoyldihydrosphingosine et une composition 4c ne faisant pas partie de l'invention contenant l'acide n-octanoyl 5-salicylique.

Ces compositions comprennent les ingrédients suivants (teneurs en % pondéral) :

| | **Exemple 4a** | **Exemple 4b** | **Exemple 4c** |
|---|---|---|---|
| Sel disodique de l'acide éthylène-diaminetétracétique | 0,15 | 0,15 | 0,15 |
| **acide n-octanoyl 5-salicylique** | **2** | - | **2** |
| Triéthanolamine | 0,9 | 0,5 | 0,8 |
| Alcool cétylique | 0,4 | 0,4 | 0,4 |
| **N-oléoyldihydrosphingosine** | 1 | 1 | - |
| N-lauroylsarcosinate d'isopropyle | 10 | 10 | 10 |
| Conservateurs | qs | qs | qs |
| Copolymère acide acryli-que/méthacrylate de stéaryle (Carbopol 1382 de chez NOVEON) | 0,5 | 0,5 | 0,5 |
| Copolymère acrylamide/acrylamido-2-methyl propane sulfonate de sodium en émulsion inverse a 40% (Simulgel 600 de chez SEPPIC) | 1 | 1 | 1 |
| Cyclohexasiloxane | 5 | 5 | 5 |
| Ethanol | 5 | 5 | 5 |
| Glycérine | 3 | 3 | 3 |
| Mélange de stéarate de glycéryle et | 0,3 | 0,3 | 0,3 |
| de PEG-100 stéarate (ARLACEL^{®} 165 FL de chez Uniqema) | | | |
| Eau | qsp 100 | qsp 100 | qsp 100 |

On a évalué les propriétés dépigmentantes de ces compositions sur un panel de 18 personnes ayant une peau asiatique. Pour chaque personne, la dose érythémale minimum (DEM) a été déterminée de façon connue.

Sur chaque avant-bras d'une personne, on a déterminé 2 zones de taille 2,25 cm² (1,5 cm X 1,5 cm).

A l'aide d'un nuancier de 52 teintes correspondant à des couleurs de peau différentes et pour lesquelles chaque paramètre de clarté C* et d'angle de teinte h dans l'espace colorimétrique CIE 1976 est quantifié, on détermine la teinte du nuancier qui se rapproche de la couleur présentée par chaque zone de l'avant-bras. On note alors la clarté C* de la couleur ainsi évaluée.

Puis on a exposé chaque zone à un rayonnement UV/SSR avec une lampe Solar Simulator de chez Oriel. On a d'abord soumis un rayonnement UV correspondant à 2 doses érythémale minimum (DEM), puis on a attendu 2 jours et selon la couleur de la zone de peau irradiée, on a ensuite soumis un rayonnement correspondant de 1,75 à 2,5 DEM. Cette opération est répétée à nouveau 2 jours après.

7 jours après la dernière irradiation UV effectuée, les zones de la peau présentent une pigmentation et on mesure à nouveau la couleur de chaque zone à l'aide du nuancier (cette mesure est donc faite à To) et on détermine la valeur de clarté C*(To) correspondante.

On commence alors le traitement des zones des avant-bras avec les compositions à tester pour évaluer leur action dépigmentante.

Sur 3 des 4 zones totales, on a appliqué une quantité de 9 µg d'une des compositions 4a, 4b, 4c (donc une seule composition est appliquée sur une zone) et la quatrième zone n'est pas traitée.

Les compositions sont ainsi appliquées 2 fois par jour (matin et soir) pendant 6 semaines.

Au bout de 6 semaines (T6) de traitement, on mesure à nouveau la couleur de chaque zone traitée à l'aide du nuancier. On détermine ainsi la valeur de la clarté correspondante C*(T6) et on la compare à la clarté C*(To) mesurée à To avant l'application des produits.

On calcule la moyenne des valeurs obtenues avant et après traitement et on les compare avec un test statistique Anova.

On a obtenu les résultats suivants :

| | **Ex 4c** | **Ex 4b** | **Ex 4a** | **zone témoin (non traitée)** | **Probabllité p : selon test Anova** |
|---|---|---|---|---|---|
| **ΔC*(T6-T0)** | -1.7 ± 1.2 | -1.8 ± 1.1 | -2.5 ± 1.1 | -0.7 ± 1.2 | ***< 0.001*** |

Les résultats obtenus sont bien significatifs puisque p < 0,001.

Les résultats obtenus montrent que la composition 4a contenant l'association de N-oléoyldihydrosphingosine et de l'acide n-octanoyl 5-salicylique permet d'obtenir un résultat d'éclaircissement de la peau plus important que ceux obtenus avec la composition 4b contenant la N-oléoyldihydrosphingosine et avec la composition 4c contenant l'acide n-octanoyl 5-salicylique.

Le résultat d'éclaircissement obtenu avec la compsoition 4b est aussi supérieur à celui obtenu avec la composition 4c.

L'association de N-oléoyldihydrosphingosine et de l'acide n-octanoyl 5-salicylique présente donc une meilleure activité dépigmentante.

## Revendications

1. Procédé cosmétique non thérapeutique pour dépigmenter et/ou éclaircir une peau présentant des taches de pigmentation comprenant l'application sur la peau d'une composition comprenant, dans un milieu physiologiquement acceptable, au moins un composé de formule (I) suivante :
R₁-CHOH-CH(NH-COR₂)(CH₂OH) (I)
dans laquelle R₁ désigne un radical alkyle en C₁₁ à C₂₁, R₂ désigne un radical hydrocarboné en C₁₁-C₁₉, linéaire, éventuellement hydroxylé et le groupe hydroxyle étant en position alpha du carbonyle, et pouvant comporter une ou plusieurs insaturations éthyléniques, notamment une ou deux insaturations éthyléniques, et au moins un agent desquamant choisi parmi l'acide salicylique et les composés de formule (II) suivante : 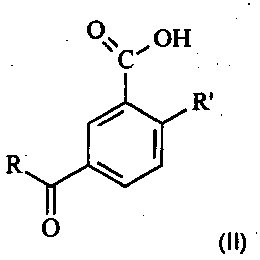 dans laquelle :
- le radical R désigne une chaîne aliphatique ayant de 2 à 22 atomes de carbone, saturée, linéaire, ramifiée ou cyclique ; une chaîne insaturée ayant de 2 à 22 atomes de carbone contenant une ou plusieurs doubles liaisons pouvant être conjuguées ; un noyau aromatique lié au radical carbonyle directement ou par l'intermédiaire de chaînes aliphatiques saturées ou insaturées ayant de 2 à 7 atomes de carbone; lesdits groupes pouvant être substitués par un ou plusieurs substituants, identiques ou différents, choisis parmi (a) les atomes d'halogène (b) le groupe trifluorométhyle, (c) des groupes hydroxyle sous forme libre ou estérifiée par un acide ayant de 1 à 6 atomes de carbone ou (d) une fonction carboxyle sous forme libre ou estérifiée par un alcool inférieur ayant de 1 à 6 atomes de carbone ;
- R' est un groupe hydroxyle ;
- ainsi que leurs sels issus d'une base minérale ou organique.

2. Procédé selon la revendication précédente, **caractérisé par le fait que** R₁ désigne un radical hydrocarboné en C₁₃-C₁₇ ; R₂ désigne un radical hydrocarboné en C₁₃-C₁₉ linéaire , éventuellement hydroxylé, et comporte éventuellement une ou plusieurs insaturations éthyléniques.

3. Procédé selon l'une quelconque des revendications précédentes, **caractérisé par le fait que** R₁ désigne un radical hydrocarboné en C₁₃-C₁₇ ; R₂ désigne soit un radical hydrocarboné en C₁₃-C₁₉ linéaire et comporte éventuellement une ou plusieurs insaturations éthyléniques, soit un radical hydrocarboné saturé en C₁₃-C₁₉ linéaire hydroxylé, le groupe hydroxyle étant en position alpha du carbonyle.

4. Procédé selon l'une quelconque des revendications précédentes, **caractérisé par le fait que** le composé de formule (I) est la N-oléoyldihydrosphingosine ou la N-2-hydroxypalmitoyldihydrosphingosine.

5. Procédé selon l'une quelconque des revendications précédentes, **caractérisé par le fait que** le radical R est un groupement alkyle en C₃-C₁₁.

6. Procédé selon l'une quelconque des revendications précédentes, **caractérisé par le fait que** la composition comprend de l'acide n-octanoyl 5-salicylique.

## Claims

1. Non therapeutic cosmetic process for depigmenting and/or lightening skin exhibiting pigmentation spots, comprising the application to the skin of a composition comprising, in a physiologically acceptable medium, at least one compound of formula (I) below:
R₁-CHOH-CH(NH-COR₂)(CH₂OH) (I)
in which R₁ denotes a C₁₁ to C₂₁ alkyl radical, and R₂ denotes a linear, optionally hydroxylated C₁₁-C₁₉ hydrocarbon-based radical, with the hydroxyl group being in the alpha-position with respect to the carbonyl, which can comprise one or more ethylenic unsaturations, in particular one or two ethylenic unsaturations, and at least one desquamating agent chosen from salicylic acid and the compounds of formula (II) below: 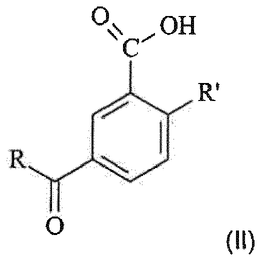 in which:
- the radical R denotes a linear, branched or cyclic, saturated aliphatic chain containing from 2 to 22 carbon atoms; an unsaturated chain containing from 2 to 22 carbon atoms containing one or more double bonds which may be conjugated; an aromatic ring linked to the carbonyl radical directly or by means of saturated or unsaturated aliphatic chains containing from 2 to 7 carbon atoms; it being possible for said groups to be substituted with one or more substituents, which may be identical or different, chosen from (a) halogen atoms, (b) a trifluoromethyl group, (c) hydroxyl groups in free form or in a form esterified with an acid containing from 1 to 6 carbon atoms, or (d) a carboxyl function in free form or in a form esterified with a lower alcohol containing from 1 to 6 carbon atoms;
- R' is a hydroxyl group;
- and also the salts thereof derived from an inorganic or organic base.

2. Process according to the preceding claim, **characterized in that** R₁ denotes a C₁₃-C₁₇ hydrocarbon-based radical; R₂ denotes a linear, optionally hydroxylated C₁₃-C₁₉ hydrocarbon-based radical and optionally comprises one or more ethylenic unsaturations.

3. Use according to either one of the preceding claims, **characterized in that** R₁ denotes a C₁₃-C₁₇ hydrocarbon-based radical; R₂ denotes either a linear C₁₃-C₁₉ hydrocarbon-based radical and optionally comprises one or more ethylenic unsaturations, or a saturated, linear, hydroxylated C₁₃-C₁₉ hydrocarbon-based radical, the hydroxyl group being in the alpha-position with respect to the carbonyl.

4. Process according to any one of the preceding claims, **characterized in that** the compound of formula (I) is N-oleoyldihydrosphingosine or N-2-hydroxypalmitoyldihydrosphingosine.

5. Process according to any one of the preceding claims, **characterized in that** the radical R is a C₃-C₁₁ alkyl group.

6. Process according to any one of the preceding claims, **characterized in that** the composition comprises 5-n-octanoylsalicylic acid.

## Patentansprüche

1. Nichttherapeutisches kosmetisches Verfahren zur Depigmentierung und/oder zum Hellermachen von Pigmentflecken aufweisender Haut, bei dem man auf die Haut eine Zusammensetzung, die mindestens eine Verbindung der folgenden Formel (I):
R₁-CHOH-CH(NH-COR₂)(CH₂OH) (I)
worin R₁ für einen C₁₁- bis C₂₁-Alkylrest steht und R₂ für einen linearen C₁₁-C₁₉-Kohlenwasserstoffrest, der gegebenenfalls hydroxyliert ist, wobei die Hydroxylgruppe in alpha-Position zur Carbonylgruppe steht, und eine oder mehrere ethylenische Ungesättigtheiten, insbesondere eine oder zwei ethylenische Ungesättigtheiten, enthalten kann, steht, und mindestens ein Entschuppungsmittel, das unter Salicylsäure und den Verbindungen der folgenden Formel (II): 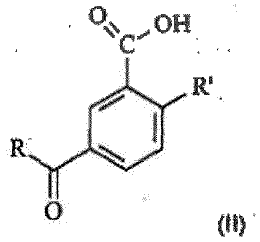 worin:
- der Rest R für eine lineare, verzweigte oder cyclische gesättigte aliphatische Kette mit 2 bis 22 Kohlenstoffatomen; eine ungesättigte Kette mit 2 bis 22 Kohlenstoffatomen und einer oder mehreren Doppelbindungen, die konjugiert sein können; oder einen aromatischen Ring, der direkt oder über gesättigte oder ungesättigte aliphatische Ketten mit 2 bis 7 Kohlenstoffatomen an den Carbonylrest gebunden ist; steht; wobei die Gruppen durch einen oder mehrere Substituenten substituiert sein können, die gleich oder verschieden sind und unter (a) Halogenatomen, (b) der Trifluormethylgruppe, (c) Hydroxylgruppen in freier Form oder mit einer Säure mit 1 bis 6 Kohlenstoffatomen veresterter Form oder (d) einer Carboxylfunktion in freier Form oder mit einem Alkohol mit 1 bis 6 Kohlenstoffatomen veresterter Form ausgewählt sind; und
- R' für eine Hydroxylgruppe steht;
- sowie Salzen davon, die sich von einer anorganischen oder organischen Base ableiten;
ausgewählt ist;
in einem physiologisch unbedenklichen Medium umfasst, aufbringt.

2. Verfahren nach dem vorhergehenden Anspruch, **dadurch gekennzeichnet, dass** R₁ für einen C₁₃- bis C₁₇-Kohlenwasserstoffrest steht und R₂ für einen linearen C₁₃-C₁₉-Kohlenwasserstoffrest, der gegebenenfalls hydroxyliert ist und gegebenenfalls eine oder mehrere ethylenische Ungesättigtheiten enthält, steht.

3. Verfahren nach einem der vorhergehenden Ansprüche, **dadurch gekennzeichnet, dass** R₁ für einen C₁₃- bis C₁₇-Kohlenwasserstoffrest steht und R₂ für einen linearen C₁₃-C₁₉-Kohlenwasserstoffrest, der gegebenenfalls eine oder mehrere ethylenische Ungesättigtheiten enthält, oder einen hydroxylierten linearen gesättigten C₁₃-C₁₉-Kohlenwasserstoffrest, wobei die Hydroxylgruppe in alpha-Position zur Carbonylgruppe steht, steht.

4. Verfahren nach einem der vorhergehenden Ansprüche, **dadurch gekennzeichnet, dass** es sich bei der Verbindung der Formel (I) um N-Oleoyldihydrosphingosin oder N-2-Hydroxypalmitoyldihydrosphingosin handelt.

5. Verfahren nach einem der vorhergehenden Ansprüche, **dadurch gekennzeichnet, dass** der Rest R für eine C₃-C₁₁-Alkylgruppe steht.

6. Verfahren nach einem der vorhergehenden Ansprüche, **dadurch gekennzeichnet, dass** die Zusammensetzung 5-n-Octanoylsalicylsäure umfasst.
